# EUROPÄISCHE PATENTSCHRIFT

(11) **EP 0 552 448 B1**
(45) Veröffentlichungstag und Bekanntmachung des Hinweises auf die Patenterteilung: **15.04.1998**
(21) Anmeldenummer: 92120668.6
(22) Anmeldetag: 03.12.1992
(51) Int. Cl.: A61K 35/78

(54) **Immunstimulierender Extrakt aus Leguminosen und Verfahren zu dessen Herstellung**
Immunostimulating leguminous extract and preparation process thereof
Extrait de légumineuse immunostimulant et son procédé de préparation

(30) Priorität: 18.12.1991 DE 4141866
(43) Veröffentlichungstag der Anmeldung: 28.07.1993
(73) Patentinhaber: MUCOS EMULSIONSGESELLSCHAFT m.b.H., D-13509 Berlin (DE)
(72) Erfinder: Ransberger, Karl, W-8022 Grünwald (DE); Piesche, Klaus, Dr., W-1000 Berlin 27 (DE)
(74) Vertreter: Grünecker, Kinkeldey, Stockmair & Schwanhäusser Anwaltssozietät

(56) Entgegenhaltungen:
- EP-A- 0 265 662
- DATABASE WPIL Week 9244, Derwent Publications Ltd., London, GB; AN 92-360698 & JP-A-4 261 122 (MORIYA H. ET AL.)

## Beschreibung

Die vorliegende Erfindung betrifft einen immunstimulierenden Extrakt aus Leguminosen und ein Verfahren zu dessen Herstellung.

Das Immunsystem ist eine biologische Funktionseinheit mit hochspezifischen Reaktionsabläufen sowohl auf humoraler wie auf zellulärer Ebene. Störungen in diesem komplexen System sind an der Entstehung zahlreicher Krankheiten ursächlich beteiligt. So führen Defekte in der Entwicklung des Immunsystems zu sogenannten Immundefektkrankheiten, die durch eine erhöhte Anfälligkeit für Infektionen und Tumoren gekennzeichnet sind, wie beispielsweise der Agammaglobulinämie. Eine gestörte immunologische Überwachungsfunktion des Organismus kann die Tumorentstehung und die Ausbreitung von pathogenen Mikroorganismen im Körper begünstigen.

Es bestehen mehrere Möglichkeiten, die Aktivität des Immunsystems zu beeinflussen. Eine seit langem bekannte Methode zur Steigerung der Aktivität des Immunsystems verwendet die Applikation definierter Antigene an einen Wirtsorganismus zur Induktion einer Immunität. Bei dieser Art der Aktivitätssteigerung des Immunsystems wird durch die Verabreichung spezieller Antigene die Reaktionsbereitschaft des Immunsystems gegen das zuvor verabreichte Antigen erhöht, so daß bei neuerlichem Eintritt des Antigens in den Organismus in kürzerer Zeit eine größere Menge Antigeninaktivierender Antikörper bereitgestellt werden. Die so erzielte Aktivitätsänderung des Immunsystems ist jedoch spezifisch auf das zur Immunisierung verwendete Antigen gerichtet und kommt nicht zum Tragen, wenn ein anderes, nicht zur vorherigen Immunisierung verwendetes Antigen in den Organismus eindringt.

In vielen Fällen ist es jedoch wünschenswert, eine generelle verbesserte Reaktivität des Immunsystems gegenüber einer Vielzahl verschiedener Antigene zu bewirken. Beispielsweise erlaubt eine generelle Immunstimulation eine verbesserte Tumorsurveillance, d.h. die Überwachungsfunktion des Immunsystems über entstehende Tumorzellen ist verbessert. Eine solche generelle Aktivitätssteigerung ist durch die Verabreichung einzelner ausgewählter Antigene nicht zu erzielen.

Aufgabe der vorliegenden Erfindung ist die Bereitstellung einer Zusammensetzung, die eine generelle immunstimulierende Wirkung besitzt.

Diese Aufgabe wird erfindunsgemäß gelöst durch einen immunstimulierenden Extrakt aus Leguminosen, erhältlich nach einem Verfahren umfassend die folgenden Schritte:
(a) epigäische Keimung von Leguminosensamen unter aseptischen Bedingungen,
(b) Frosten und Auftauen der Keimlinge,
(c) Homogenisieren der aufgetauten Keimlinge mit Wasser,
(d) Abtrennen der wäßrigen Phase von unlöslichen Partikeln,
(e) Ausfällen der wirkstoffhaltigen Fraktion aus der wäßrigen Phase durch Zusatz eines organischen Lösungsmittels, das mit Wasser mischbar ist, und
(f) Herstellen eines Trockenextraktes aus der ausgefällten Fraktion in bekannter Weise.

Eine weitere Aufgabe der Erfindung ist es, ein Verfahren zur Herstellung des erfindungsgemäßen immunstimulierenden Extraktes zur Verfügung zu stellen.

Diese Aufgabe wird erfindungsgemäß durch das obige Verfahren gelöst.

Es sind mehrere Verfahren zur Herstellung von Leguminosenextrakten bekannt, wobei diese Verfahren darauf abzielen, einzelne Bestandteile von Leguminosen derart anzureichern, daß diese einer biochemischen Charakterisierung zugänglich sind.

Die Aufarbeitung von gekeimten Erbsensamen zur Isolierung und Charakterisierung von darin enthaltenen proteolytischen Enzymen ist beschrieben in C.-E. Danielsson, Acta Chem. Scand., 5, 791-804, 1951. Die Aufbereitung von ungekeimten Erbsensamen zur Isolierung und Reinigung einer Protease wird in R. Soedigo und M. Gruber, Biochem. Biophys. Acta 44, 315-323, 1960 beschrieben. Weitere Verfahren sind bekannt aus J.L. Young und J.E. Varner, Arch. Biochem. Biophys., 84, 71-78, 1959 und S.M.M. Basha und L. Beevers, Planta, 124(1), 77-87, 1975. Die bekannten Herstellungsverfahren von Leguminosenextrakten dienen zur Isolierung und Reinigung von proteolytischen Enzymen, wobei die bekannten Extrakte keine immunstimulierende Wirkung besitzen.

Der vorliegende erfindungsgemäße Extrakt wird vorzugsweise hergestellt aus einem Mitglied der Familie Papilionaceae, die zur Ordnung der Leguminosen zählt. Besonders bevorzugt ist ein Extrakt, der aus einem Mitglied der Unterfamilie Faboideae, insbesondere der Gattungen Lens, Pisum, Cicer, Phaseolus, Lupinus, Vicia, Onobrychis, Medicago oder Trifolium, hergestellt worden ist.

Ganz besonders geeignet zur Herstellung des erfindungsgemäßen Extrakts sind Lens culinaris (Speiselinsen), Pisum sativum (Gemüsesaaterbsen), Cicer arietinum (Kichererbsen) oder Phaseolus radiatus (Mungbohnen). Diese in der gärtnerischen oder landwirtschaftlichen Nahrungsgüterproduktion verwendeten Leguminosen sind besonders arm an bzw. frei von biogenen Toxinen, wie z.B. cyanogenen Glykosiden oder Chinolizidin-Alkaloiden.

Vorzugsweise erfolgt bei der Herstellung der erfindungsgemäßen Extrakte die epigäische Keimung für einen Zeitraum von ungefähr einem bis acht Tagen, wobei die Keimung für eine Dauer von ungefähr 3 Tagen besonders bevorzugt ist.

Zur Gewährleistung der aseptischen Keimungsbedingungen werden die trockenen Leguminosesamen vor der Keimung zunächst desinfiziert, vorzugsweise unter Verwendung von 70-%igem Isopropanol. Anschließend werden die desinfizierten Samen unter den aseptischen Bedingungen zum Quellen und Keimen gebracht. Das Keimen erfolgt vorzugsweise unter im Beispiel 3 näher beschriebenen Bedingungen.

Erfolgt die Keimung nicht unter aseptischen Bedingungen, zeigen die erhaltenen Extrakte zwar eine immunstimulierende Wirkung, diese wird jedoch von unerwünschten Nebenwirkungen begleitet, welche auf mikrobielle Kontaminationen zurückzuführen sind.

Die Verwendung von demineralisiertem Wasser bei dem Homogenisieren der aufgetauten Keimlinge ist besonders vorteilhaft.

Die Abtrennung der wäßrigen Phase von festen Bestandteilen nach dem Homogenisierungsvorgang erfolgt vorzugsweise durch Zentrifugation.

Zum Ausfällen der wirkstoffhaltigen Fraktion aus der nach der Zentrifugation erhaltenen wäßrigen Phase wird vorzugsweise das drei- bis zehnfache Volumen der wäßrigen Phase eines organischen Lösungsmittels verwendet. Besonders gute Ergebnisse werden erzielt, wenn das Lösungsmittel in einem vierfachen Überschuß zu dem Volumen der wäßrigen Phase erfolgt. Ein organisches Lösungsmittel, das mit Wasser mischbar ist, wie z.B. Äthylalkohol, Isopropylalkohol oder Aceton, kann verwendet werden, wobei Aceton besonders bevorzugt ist.

Die Herstellung eines Trockenextraktes aus der ausgefällten Fraktion erfolgt in bekannter Weise, wie beispielsweise die Gewinnung von Enzympräparaten in Form von Trockenpulvern, beschrieben in Ullmanns Encyklopädie der technischen Chemie, Band 10, S.475-561, 1975.

Ohne an eine Theorie gebunden zu sein, der erfindungsgemäße Extrakt entfaltet seine immunstimulierende Wirkung vermutlich aufgrund der in dem Extrakt vorhandenen proteolytisch aktiven Bestandteile. Diese proteolytische Aktivität des Extraktes ist vermutlich für die makrophagenstimulierende Wirkung des Extraktes verantwortlich, wobei die erhöhte Aktivität der Makrophagen ihrerseits vermutlich die beobachtete generelle Immunstimulation mitverursacht.

Die Bestimmung der proteolytischen Aktivität der Extrakte erfolgte gemäß der von M. Kunitz, in J. Gen. Physiol., 30, 291, 1947 beschriebenen Kaseinmethode unter leicht modifizierten Bedingungen. Die exakte Durchführung der Proteinasebestimmung wird unten näher beschrieben.

Die folgenden Beispiele erläutern die Erfindung.

Die im folgenden aufgeführten Reagenzien sind alle gewerblich erhältlich.

### Beispiel 1

Bestimmung der proteolytischen Aktivität in den hergestellten Extrakten.

Das verwendete Verfahren basiert auf der von M. Kunitz, in J. Gen. Physiol., 30, 291, 1947 beschriebenen Kaseinmethode.

Eine Lösung von 37,5 mg Kasein in 2,5 ml 0,05 M TRIS/ HCL, pH 7,5 wurde für 10 Minuten bei 35° C im Wasserbad temperiert. Anschließend wurden 0,5 ml Extraktlösung in 0,05 M TRIS/HCL, pH 7,5 der Kaseinlösung zugesetzt. Nach Inkubation für 10 Minuten wurden 3 ml 10 %-ige Trichloressigsäurelösung zugesetzt. Nach Zentrifugation bei 5000 Upm und Filtration über einen Papierfilter erfolgte die Messung der Absorption bei 280 nm.

Eine CU (kaseinolytische Einheit) entspricht der Extraktmenge, die unter den oben angegebenen Bedingungen eine Extinktionszunahme von 1,000 bewirkt.

### Beispiel 2

Bestimmung der immunstimulierenden Aktivität von Leguminosenextrakten.

Als Indikator für die Aktivität der hergestellten Leguminosenextrakte diente die Induktion von Lymphokinen, insbesondere des Tumornekrosefaktors (TNF), nach Inkubation von Makrophagen mit den hergestellten Leguminosenextrakten. Die Bestimmung wurde im einzelnen wie folgt durchgeführt:

Humanblut von gesunden Spendern wurde über Lymphoprep (erhältlich von Nycomed, Norwegen) getrennt, die mononukleären Zellen wurden zweimal mit Phosphatpuffer gewaschen und anschließend in RPMI 1640 Medium, ergänzt mit 25 mM Hepes, Penizillin (100 I.E./ml), Streptomyzin (0,1 mg/ml), Glutamin (10 mg/ml) und hitzeinaktiviertem 10 % fötalem Kälberserum, aufgenommen. Die Zellen wurden mit der Extraktlösung auf eine Endkonzentration von 1 x 10⁶ Zellen/ml eingestellt (Verhältnis 40 pg Extrakt/Zelle). Die gesamte Inkubationszeit betrug 48 Stunden. In Zeitintervallen von 4 Stunden wurden dem Inkubationsansatz Proben zur Bestimmung der TNF-Menge entnommen. Die Bestimmung von TNF erfolgte über die cytotoxische Aktivität der Überstände auf die transformierte Mausfibroblastenlinie L-929, die aus CH3 Mäusen stammt.

Als Kontrolle wurde die direkte Toxizität der Extraktlösungen auf die L-929 Zellen selbst gemessen. In allen Fällen zeigte der Extrakt selbst keine Toxizität.

Eine immunstimulierende Aktivität lag vor, wenn mit dieser Bestimmungsmethode TNF nachgewiesen werden konnte.

### Beispiel 3

Herstellung eines Extraktes aus Lens culinaris.

1000 g grüne Speiselinsen (Lens culinaris), bezogen von der Firma Rapunzel Naturkost, Legau, wurden 5 Minuten in 70 % Isopropanol desinfiziert, und anschließend wurden die Linsen mit demineralisiertem Wasser gewaschen. Anschließend wurden die Linsen über Nacht mit reichlich demineralisiertem, abgekochtem Wasser bei Raumtemperatur quellengelassen. Die Betthöhe der Samen sollte dabei 10 cm nicht überschreiten. Das Gewicht der Linsen nach dem Quellen betrug 2028 g. Nach dem Abtropfen auf einem Sieb wurde das gequollene Material auf eine mit sterilem Filterpapier ausgelegte sterile Stahlschale (80 x 30 cm) in einfacher Schicht ausgebreitet, mit einer Frischhaltefolie aus Polyethylen überzogen und anschließend einer epigäischen Keimung bei Raumtemperatur unter dem Einfluß von indirektem Tageslicht unter sterilen Bedingungen innerhalb einer Sicherheitswerkbank für 72 Stunden unterzogen. Dabei wurde das Filterpapier ständig feuchtgehalten. Das Gewicht des gekeimten Materials betrug am Ende der Keimung 2040 g. Die Linsenkeimlinge wurden danach für die Dauer von 24 Stunden bei -30° C eingefroren und anschließend wieder aufgetaut. Die noch kalte Masse der Keimlinge wurde mit 4 l kaltem, demineralisiertem Wasser versetzt und unter hochtourigen Bedingungen mit einem Ultra-Turrax Typ T 45 DX (erhältlich von der Firma Janke & Kunkel, Staufen) homogenisiert, wobei zunächst für 7 Minuten der Generator TP 45/2G und anschließend weitere 7 Minuten der Generator TP 45/6G verwendet wurde. Das Homogenat wurde für 60 Minuten bei 5000 Upm bei +6° C zentrifugiert. Der Überstand mit einem Volumen von 4,44 l wurde unter mechanischem Rühren (ungefähr 100 Upm) mit dem vierfachen Volumen Aceton, das auf -30° C vorgekühlt worden war, versetzt. Die entstandene Fällung wurde nach einer Stehzeit von 1 Stunde und nach vorherigem Entfernen des klaren Überstandes mit Hilfe einer Fritte (Glassinternutsche) mit der Porosität 4 abgetrennt. Der auf dem Frittenboden gesammelte Filterkuchen wurde mehrfach unter vorsichtigem Rühren mit Aceton gewaschen und durch Vakuum abgesaugt. Nach Vortrocknen an der frischen Luft und Endtrocknen in einem Exsikkator wurde ein feines, amorphes, cremefarbenes Pulver mit arteigenem Geruch erhalten. Die Ausbeute betrug 126,93 g.

Der erhaltene Trockenextrakt ergab eine proteolytische Aktivität von 19,33 CU/g. 40 µg des Extraktes bewirkten eine TNF-Freisetzung von 70 ng, bestimmt mit der vorstehend beschriebenen Methode. Die freigesetzte TNF-Menge ist ein Indikator für die immunstimulierende Aktivität des Extraktes.

### Beispiel 4

Herstellung eines immunstimulierenden Extraktes aus Pisum sativum.

50 g trockene Gemüsesaaterbsen (Pisum sativum) der Sorte Markerbsen "Lancet", erhältlich bei der Firma Samen Schmitz, Aschheim, wurden wie in Beispiel 1 aufgearbeitet. Die Ausbeute betrug 4,19 g.

Die proteolytische Aktivität des erhaltenen Extraktes betrug 10,3 CU/g. 40 µg des Extraktes bewirkten eine TNF-Freisetzung von 42 ng, bestimmt mit der vorstehend beschriebenen Methode.

Wie aus den vorherigen Erläuterungen ersichtlich, besitzt der erfindungsgemäße Extrakt einen deutlichen makrophagenstimulierenden, antigenunabhängigen Effekt und eignet sich somit zur Verwendung als generelles immunstimulierendes Mittel. Insbesondere ist der erfindungsgemäße Extrakt in der Lage, die sogenannte Tumor-Surveillance, d.h. die Überwachungsfunktion des Immunsystems über entstehende Tumorzellen, deutlich zu verbessern.

## Patentansprüche

1. Verfahren zur Herstellung eines immunstimulierenden Extraktes aus Leguminosen, umfassend die folgenden Schritte:
(a) epigäische Keimung von Leguminosensamen unter aseptischen Bedingungen,
(b) Frosten und Auftauen der Keimlinge,
(c) Homogenisieren der aufgetauten Keimlinge mit Wasser,
(d) Abtrennen der wäßrigen Phase von unlöslichen Partikeln,
(e) Ausfällen der wirkstoffhaltigen Fraktion aus der wäßrigen Phase durch Zusatz eines organischen Lösungsmittels, das mit Wasser mischbar ist, und
(f) Herstellen eines Trockenextraktes aus der ausgefällten Fraktion in bekannter Weise.

2. Verfahren nach Anspruch 1, **dadurch gekennzeichnet**, daß der Extrakt aus mindestens einem Mitglied der Familie Papilionaceae hergestellt wird.

3. Verfahren nach Anspruch 2, **dadurch gekennzeichnet**, daß der Extrakt aus mindestens einem Mitglied der Gattung Lens, Pisum, Cicer, Phaseolus, Lupinus, Vicia, Onobrychis, Medicago oder Trifolium hergestellt wird.

4. Verfahren nach einem der Ansprüche 1 bis 3, **dadurch gekennzeichnet**, daß die epigäische Keimung in Schritt (a) für eine Dauer von ungefähr einem bis acht Tagen erfolgt.

5. Verfahren nach Anspruch 4, **dadurch gekennzeichnet**, daß die epigäische Keimung für eine Dauer von ungefähr drei Tagen erfolgt.

6. Verfahren nach einem der Ansprüche 1 bis 5, **dadurch gekennzeichnet**, daß das Homogenisieren in Schritt (c) mit demineralisiertem Wasser erfolgt.

7. Verfahren nach einem der Ansprüche 1 bis 6, **dadurch gekennzeichnet**, daß das Abtrennen der wäßrigen Phase in Schritt (d) durch Zentrifugation erfolgt.

8. Verfahren nach einem der Ansprüche 1 bis 7, **dadurch gekennzeichnet**, daß das Ausfällen der wirkstoffhaltigen Fraktion in Schritt (e) durch Zusatz des organischen Lösungsmittels in drei- bis zehnfachem Überschuß zu der wäßrigen Phase erfolgt.

9. Verfahren nach Anspruch 8, **dadurch gekennzeichnet**, daß das Ausfällen durch Zusatz eines vierfachen Überschusses erfolgt.

10. Verfahren nach einem der Ansprüche 1 bis 9, **dadurch gekennzeichnet**, daß das Ausfällen der wirkstoffhaltigen Fraktion durch Zusatz von Aceton erfolgt.

11. Immunstimulierender Extrakt aus Leguminosen, erhältlich nach einem der Verfahren gemäß Ansprüchen 1 bis 10.

12. Verwendung des Extrakts nach Anspruch 11 zur Herstellung eines Medikaments zur Stimulierung des Immunsystems.

## Claims

1. A process for the preparation of an immunostimulating extract of leguminous plants, comprising the following steps:
(a) epigeous germination of seeds of leguminous plants under aseptic conditions,
(b) freezing and thawing of the embryos,
(c) homogenisation of the thawed embryos with water,
(d) separation of the aqueous phase from insoluble particles,
(e) precipitation from the aqueous phase of the fraction containing active substances by the addition of an organic solvent which is miscible with water, and
(f) preparation in known manner of a dry extract from the fraction precipitated out.

2. A process according to claim 1, **characterised in that** the extract is prepared from at least one member of the family Papilionaceae.

3. A process according to claim 2, **characterised in that** the extract is prepared from at least one member of the species Lens, Pisum, Cicer, Phaseolus, Lupinus, Vicia, Onobrychis, Medicago or Trifolium.

4. A process according to one of claims 1 to 3, characterised in that the epigeous germination in step (a) takes place for a period of about one to eight days.

5. A process according to claim 4, **characterised in that** the epigeous germination takes place for a period of about three days.

6. A process according to one of claims 1 to 5, **characterised in that** the homogenisation in step (c) is carried out using demineralised water.

7. A process according to one of claims 1 to 6, **characterised in that** the separation of the aqueous phase in step (d) is carried out by centrifugation.

8. A process according to one of claims 1 to 7, **characterised in that** the precipitation of the fraction containing active substances in step (e) is carried out by the addition of the organic solvent in threefold to tenfold excess relative to the aqueous phase.

9. A process according to claim 8, **characterised in that** the precipitation is carried out by the addition of a fourfold excess.

10. A process according to one of claims 1 to 9, **characterised in that** the precipitation of the fraction containing active substances is carried out by the addition of acetone.

11. Immunostimulating extract of leguminous plants, obtainable by one of the processes according to claims 1 to 10.

12. The use of the extract according to claim 11 for the preparation of a medicament for stimulating the immune system.

## Revendications

1. Procédé pour la préparation d'un extrait immunostimulant de légumineuses, comprenant les étapes suivantes:
(a) germination épigée de graines de légumineuses dans des conditions aseptiques,
(b) congélation et décongélation des germes,
(c) homogénéisation avec de l'eau des germes décongelés,
(d) séparation de la phase aqueuse d'avec les particules insolubles,
(e) précipitation de la fraction contenant les substances actives, à partir de la phase aqueuse, par addition d'un solvant organique miscible à l'eau, et
(f) préparation d'un extrait sec à partir de la fraction précipitée, d'une façon connue.

2. Procédé selon la revendication 1, caractérisé en ce que l'extrait est préparé à partir d'au moins un membre de la famille des papilionacées.

3. Procédé selon la revendication 2, caractérisé en ce que l'extrait est préparé à partir d'au moins un membre du genre *Lens*, *Pisum*, *Cicer*, *Phaseolus*, *Lupinus*, *Vicia*, *Onobrychis*, *Medicago* ou *Trifolium*.

4. Procédé selon l'une des revendications 1 à 3, caractérisé en ce que la germination épigée dans l'étape (a) s'effectue pendant une durée d'environ un à huit jours.

5. Procédé selon la revendication 4, caractérisé en ce que la germination épigée s'effectue pendant une durée d'environ trois jours.

6. Procédé selon l'une des revendications 1 à 5, caractérisé en ce que l'homogénéisation dans l'étape (c) est effectuée avec de l'eau déminéralisée.

7. Procédé selon l'une des revendications 1 à 6, caractérisé en ce que la séparation de la phase aqueuse dans l'étape (d) est effectuée par centrifugation.

8. Procédé selon l'une des revendications 1 à 7, caractérisé en ce que la précipitation de la fraction contenant les substances actives, dans l'étape (e), est effectuée par addition du solvant organique en un excès de trois à dix fois par rapport à la phase aqueuse.

9. Procédé selon la revendication 8, caractérisé en ce que la précipitation est effectuée par addition d'un excès de quatre fois.

10. Procédé selon l'une des revendications 1 à 9, caractérisé en ce que la précipitation de la fraction contenant les substances actives est effectuée par addition d'acétone.

11. Extrait immunostimulant de légumineuses, pouvant être obtenu selon l'un des procédés conformes aux revendications 1 à 10.

12. Utilisation de l'extrait selon la revendication 11, pour la fabrication d'un médicament destiné à la stimulation du système immunitaire.
